# EUROPEAN PATENT APPLICATION

(11) **EP 1 977 770 A1**
(43) Date of publication of application: **08.10.2008**
(21) Application number: 07251371.6
(22) Date of filing: 29.03.2007
(51) Int. Cl.: A61L 9/12, A61L 9/14, A61L 9/03, G04B 25/00

(54) **Timer controlled olfactory fragrance dispenser**

(71) Applicant: Tai-Her, Yang, Si-Hu Town Dzan-Hwa (TW)
(72) Inventor: Tai-Her, Yang, Si-Hu Town Dzan-Hwa (TW)
(74) Representative: Pratt, David Martin

(57) **Abstract**

A timer controlled olfactory fragrance dispenser that has the olfaction as the interface to aid time telling while the conventional clock works on the sense of hearing and/or sight as the interface for time telling; providing additional funs and sense of comfort, the present invention allows its user to "smell" the time when he/she is in the dark or in sleeping, and the blind and/or the deaf to "smell" the changes of time; and allowing to be preset for emitting fragrance of different odors, the lapse between two emissions or the concentration of the fragrant; and may be made in a portable form to satisfy personal needs.

## Description

### BACKGROUND OF THE INVENTION

### (a) Field of the Invention

The present invention is related to a timer controlled olfactory fragrance dispenser, and more particularly to one that functions as a standing-alone fragrance dispenser capable of emitting different odors of fragrance or as a timer having the same or different fragrance detectable by the smell sense as the time telling interface instead of the conventional timer that has visual or audible sense as the time telling interface.

### (b) Description of the Prior Art:

The conventional intermission type of air fragrance dispenser is found with the flaws that it emits the same odor of fragrance that eventually becomes not fragrant at all after a given time when people are get used to smell it; and that it remains emitting during night hours to cause wastes when it is installed in an office or a public place. The conventional timer tells time depending on the passive receiving by the aural sense of the user or the active watching on the hands, scales, or digital display by the vision sense of the user as the interface when the audible sounds of clock is prevented in an environment demanding absolute silence. Furthermore, the conventional time telling method fails to satisfy the blind and/or the deaf the sense to detect changes in time. In the present invention provides same or different odors as the interface to tell time, or functions as a fragrance dispenser that emits different detectable odors.

### SUMMARY OF THE INVENTION

The primary purpose of the present invention is to provide a timer controlled olfactory fragrance dispenser that has the olfaction as the interface to aid time telling while the conventional clock works on the sense of hearing and/or sight as the interface for time telling. While providing additional funs and sense of comfort, the present invention allows its user to "smell" the time when he/she is in the dark or in sleeping, and the blind and/or the deaf to "smell" the changes of time. The present invention is a breakthrough to correct the flaws of the prior art that works day and night and intermittently only a single odor by allowing to be preset for emitting fragrance of different odors, the lapse between two emissions or the concentration of the fragrant. Another advantage yet of the present invention is that it may be made in a portable form to satisfy personal needs.

### BRIEF DESCRIPTION OF THE DRAWINGS

Fig. 1 is a block chart of a timer controlled olfactory fragrance dispenser of the present invention.
Fig. 2 is a block chart of the timer controlled olfactory fragrance dispenser of the present invention wherein the present invention functions as a traditional timer using the measuring unit of the standard time telling device to provide olfactory time telling function.
Fig. 3 is a block chart of the timer controlled olfactory fragrance dispenser of the present invention wherein the present invention functions as a traditional timer using the measuring unit of the standard time telling device to provide olfactory time telling function incorporated with conventional time telling function via sense of hearing and vision.

### DETAILED DESCRIPTION OF THE PREFERRED EMBODIMENTS

The present invention is related to a timer controlled olfactory fragrance dispenser. It can be preset to emit fragrance of different odors while providing combined functions as a clock and a fragrance dispenser. It functions as a conventional clock to tell time in the standard unit of time telling and to emit the fragrance of same or different odor at a scheduled time table as an aid of telling the time via olfaction.

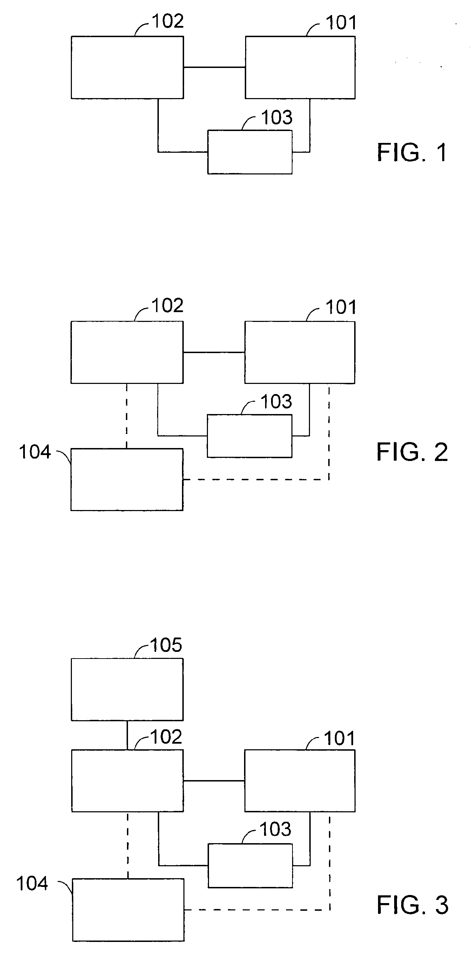

Referring to Fig. 1 for a block chart of a timer controlled olfactory fragrance dispenser of the present invention, other than necessarily optional the casing and the general manual operating device, the present invention is essentially comprised of the followings:
-- a controllable odor supply unit 101: comprised of two or more than two different olfactory odor supply sources in solid, liquid or gaseous state that are emitted either by itself, by heating, or by blowing, and is controlled by electric or mechanical power to be emitted into the designated space or not; an optional unit to set up and control the concentration of the odor may be disposed to the controllable odor supply unit 101;
-- a timing control device 102: related to a digital or analogy time output control device comprised of a mechanical, dynamo-mechanical or solid-state electronic timing device that allows to set up the operation time so that when the preset time is arrived at, the electric or mechanical power is outputted for the control of the controllable odor supply unit 101 to emit the odors for the session as preset; and
-- an odor supply energy driver 103: to supply mechanical or electric power to the controllable odor supply unit 101 if the timing control device 102 is already provided with a local energy driver; or vice versa.

In the timer controlled olfactory fragrance dispenser described above, as preset by the timing control device 102, the controllable odor supply unit 101 continuously or intermittently emit olfactory odors in different sessions in sequence.

Furthermore, an optional manual control device is adapted to the timer controlled olfactory fragrance dispenser for the purpose of controlling the controllable odor supply unit 101 to emit the odor or not and which type of odor to be emitted.

Fig. 2 is a block chart of the timer controlled olfactory fragrance dispenser of the present invention wherein the present invention functions as a traditional timer using the measuring unit of the standard time telling device to provide olfactory time telling function. The timing control device 102 functions as a conventional clock using one or multiple time telling measurement unit of the prior art including hour, half an hour, quarter, minute, second, day, night, month, season, and year as the time unit that can be preset by the timing control device 102. Same olfactory odor or different odors continuously or intermittently is emitted upon the preset time is arrived at so to actively tell the time by odor. The preferred embodiment of the present invention illustrated in Fig. 2 is essentially comprised of the controllable odor supply unit 101, the timing control device 102, the odor supply energy driver 103, and a setting up device 104. Wherein, the electric or mechanical power form the odor supply energy driver 103 drives the timing control device 102 and the controllable odor supply unit 101. As preset by the setting up device 104, the timing control device 102 engages in the standard operation to tell the time; or alternatively the timing control device 102 operates as a clock of the prior art on its on. The time telling measurement unit of the standard time telling device of the prior art is used to set up the time in the present invention to control the controllable odor supply unit 101 in emitting olfactory odor to aid in telling the time or controlling the type of the odor emitted.

The preferred embodiment illustrated in Fig. 2 is capable of functioning as a clock of the prior art to tell the time using the measurement unit of telling the time found with a standard time telling device of the prior art to become a time telling device by emitting olfactory odor. The controllable odor supply unit 101 may cause to emit the same odor or different odors as preset by the setting up device 104.

Fig. 2 is a block chart of the timer controlled olfactory fragrance dispenser of the present invention wherein the present invention functions as a traditional timer using the measuring unit of the standard time telling device to provide olfactory time telling function. Other than the casing and the general manual operating device selected as required, the preferred embodiment of the present invention is essentially comprised of the followings:
-- a controllable odor supply unit 101: comprised of one or more than one same or different olfactory odor supply sources in solid, liquid or gaseous state that are emitted either by itself, by heating, or by blowing, and is controlled by electric or mechanical power to be emitted into the designated space or not; an optional unit to set up and control the concentration of the odor may be disposed to the controllable odor supply unit 101;
-- a timing control device 102: related to a digital or analogy time output control device comprised of a mechanical, dynamo-mechanical or solid-state electronic timing device that allows to set up the operation time so that when the preset time is arrived at, the electric or mechanical power is outputted for the control of the controllable odor supply unit 101 to emit the odors for the session as preset;
-- an odor supply energy driver 103: to supply mechanical or electric power to the controllable odor supply unit 101 if the timing control device 102 is already provided with a local energy driver; or vice versa; and
-- a setting up device 104: related to a mechanical, dynamo-mechanical, or solid-state electronic device provided for setting up the controllable odor supply unit 101 to control the control timing of the timing control device 102 using the time telling unit of the prior art, or control the type of odor and the concentration of the olfactory odor emitted from timing control device 102, or set up the operation mechanisms of the controllable odor supply unit 101 and the timing control device 102 at the same time.

The functional construction of telling the time by emitting olfactory odor of the time controlled olfactory odor dispenser described above functions as a clock of the prior art having the time telling measurement unit of the standard time telling device as the preset time telling unit so to control any or all of the following functions:
(1) Set up the controllable odor supply unit 101 to function as a clock in relation to the timing control device 102 for setting up the time telling operation using the time telling measurement unit of the standard time telling device as the unit to set up time telling; to control the source of olfactory odor to emit the same odor or different odors; or to control the intensity of the odor; or provide the session of continuous or intermittent emission of the olfactory odor;
(2) to set up point of time for the timing control device 102 to control the controllable odor supply unit 101 using the time telling measurement unit of the standard time telling device as the unit to set up time telling as described in subparagraph (1); or
(3) Both functions respectively described in subparagraphs (1) and (2) are provided at the same time.

Furthermore, in the time controlled olfactory odor dispenser functions as a clock of the prior art having the time telling measurement unit of the standard time telling device as the present time telling unit to provide the function of telling the time by emitting olfactory odor, an optional manual control device is adapted to the timer controlled olfactory fragrance dispenser for the purpose of controlling the controllable odor supply unit 101 to emit the odor or not and which type of odor to be emitted.

Fig. 3 is a block chart of the timer controlled olfactory fragrance dispenser of the present invention wherein the present invention functions as a traditional timer using the measuring unit of the standard time telling device to provide olfactory time telling function incorporated with conventional time telling function via sense of hearing and vision. Wherein, a conventional audio or video display unit 105 is further incorporated into the preferred embodiment illustrated in Fig. 2 while the timing device functions as a clock of the prior art and is set to be adapted with the time telling measurement unit of a standard time telling device using any of or any combination of the units including hour, half an hour, quarter, minute, second, day, night, month, season, and year to control the timing of the controllable odor supply unit 101 to emit olfactory odor for providing supplementary active time telling function in facilitating its use. Other than necessarily optional the casing and the general manual operating device, the preferred embodiment of the present invention as illustrated in Fig. 3 is essentially comprised of the followings:
-- a controllable odor supply unit 101: comprised of one or more than one same or different olfactory odor supply sources in solid, liquid or gaseous state that are emitted either by itself, by heating, or by blowing, and is controlled by electric or mechanical power to be emitted into the designated space or not; an optional unit to set up and control the concentration of the odor may be disposed to the controllable odor supply unit 101;
-- a timing control device 102: related to a digital or analogy time output control device comprised of a mechanical, dynamo-mechanical or solid-state electronic timing device that allows to set up the operation time so that when the preset time is arrived at, the electric or mechanical power is outputted for the control of the controllable odor supply unit 101 to emit the odors for the session as preset;
-- an odor supply energy driver 103: to supply mechanical or electric power to the controllable odor supply unit 101 if the timing control device 102 is already provided with a local energy driver; or vice versa;
-- a setting up device 104: related to a mechanical, dynamo-mechanical, or solid-state electronic device provided for setting up the controllable odor supply unit 101 to control the control timing of the timing control device 102 using the time telling unit of the prior art, or control the type of odor and the concentration of the olfactory odor emitted from timing control device 102, or set up the operation mechanisms of the controllable odor supply unit 101 and the timing control device 102 at the same time; and
-- an audio or video display unit 105: related to audio device adapted to a timer comprised of conventional mechanical or electronic circuit including active time telling means including loudspeaker of a mechanical audio, or electronic device; or a video display device including a passive time telling device comprised of mechanical hands, jotting figures or electronic display.

The functional construction of telling the time by emitting olfactory odor of the time controlled olfactory odor dispenser described above incorporated with conventional time telling functions of the conventionally audio and video time telling functions as a clock of the prior art having the time telling measurement unit of the standard time telling device as the preset time telling unit so to control any or all of the following functions:
(1) Set up the controllable odor supply unit 101 to function as a clock in relation to the timing control device 102 for setting up the time telling operation using the time telling measurement unit of the standard time telling device as the unit to set up time telling; to control the source of olfactory odor to emit the same odor or different odors; or to control the intensity of the odor; or provide the session of continuous or intermittent emission of the olfactory odor; and is at the same time provided with an audio device adapted to a timer comprised of conventionally mechanical or electronic circuit including active time telling means including loudspeaker of a mechanical audio, or electronic device; or a video display device including a passive time telling device comprised of mechanical hands, jotting figures or electronic display;
(2) to set up point of time for the timing control device 102 to control the controllable odor supply unit 101 using the time telling measurement unit of the standard time telling device as the unit to set up time telling as described in subparagraph (1); or
(3) Both functions respectively described in subparagraphs (1) and (2) are provided at the same time.

For the functional construction of telling the time by emitting olfactory odor of the time controlled olfactory odor dispenser incorporated with conventional time telling functions of the conventionally audio and video time telling, an optional manual control device is adapted to the timer controlled olfactory fragrance dispenser for the purpose of controlling the controllable odor supply unit 101 to emit the odor or not and which type of odor to be emitted.

For both preferred embodiments of the present invention as illustrated in Figs. 2 and 3, the time controlled device operates as a clock of the prior art, and uses any of or any combination of the universal timing system including hour, half an hour, quarter, minute, second, week, month, season, or year as its time telling unit to control whether the same odor or different odors, concentration of odor, and timing of the olfactory odor to be emitted from the controllable odor supply unit 101.

It should be noted that the time controlled olfactory odor supply device of the present invention within the scope of its basic working principles may be provided in desktop, wall mounting or portable fragrance dispenser adapted to a timer or not.

Whereas the conventional timer either actively made it sensible by the hearing sense of the man or passively made it be seen by hands, jotting figures or a display unit, the present invention creates the interface of time telling by emitting the same odor or different odors olfactory to human smell sense as an active aid in telling the time. The present invention while providing more fun and comforts for the use allows the blind and/or the deaf to be able to sense the changes in time. Furthermore, the present invention preset emission of same or different odors during the different sessions at different concentration as desired, and it made be made in a portable form to meet personalized needs. Therefore, the present invention is innovative and with specific functions, and this application for a patent is filed accordingly.

## Claims

1. A timer controlled olfactory fragrance dispenser allows to be preset to emit fragrance of different odors while providing combined functions as a clock and a fragrance dispenser; functions as a conventional clock to tell time in the standard unit of time telling and to emit the fragrance of same or different odor at a scheduled time table as an aid of telling the time via olfaction.

2. The timer controlled olfactory fragrance dispenser of Claim 1, wherein, other than necessarily optional the casing and general manual operation device, the preferred embodiment of the present invention is essentially comprised of the following:
-- a controllable odor supply unit 101: comprised of two or more than two different olfactory odor supply sources in solid, liquid or gaseous state that are emitted either by itself, by heating, or by blowing, and is controlled by electric or mechanical power to be emitted into the designated space or not; an optional unit to set up and control the concentration of the odor may be disposed to the controllable odor supply unit 101;
-- a timing control device 102: related to a digital or analogy time output control device comprised of a mechanical, dynamo-mechanical or solid-state electronic timing device that allows to set up the operation time as required so that when the preset time is arrived at, the electric or mechanical power is outputted for the control of the controllable odor supply unit 101 to emit the odors for the session as preset; and
-- an odor supply energy driver 103: to supply mechanical or electric power to the controllable odor supply unit 101 if the timing control device 102 is already provided with a local energy driver; or vice versa;
In the timer controlled olfactory fragrance dispenser described above, as preset by the timing control device 102, the controllable odor supply unit 101 continuously or intermittently emit olfactory odors in different sessions in sequence.

3. The timer controlled olfactory fragrance dispenser of Claim 2, wherein, an optional manual control device is adapted to the timer controlled olfactory fragrance dispenser for the purpose of controlling the controllable odor supply unit 101 to emit the odor or not and which type of odor to be emitted.

4. The timer controlled olfactory fragrance dispenser of Claim 1, wherein, it is capable of functioning as a clock of the prior art to tell the time using the measurement unit of telling the time found with a standard time telling device of the prior art to become a time telling device by emitting olfactory odor. The controllable odor supply unit 101 may cause to emit the same odor or different odors as preset by the setting up device 104; and other than necessarily optional the casing and the general manual operating device, the preferred embodiment of the present invention is essentially comprised of the followings:
-- a controllable odor supply unit 101: comprised of one or more than one same or different olfactory odor supply sources in solid, liquid or gaseous state that are emitted either by itself, by heating, or by blowing, and is controlled by electric or mechanical power to be emitted into the designated space or not; an optional unit to set up and control the concentration of the odor may be disposed to the controllable odor supply unit 101;
-- a timing control device 102: related to a digital or analogy time output control device comprised of a mechanical, dynamo-mechanical or solid-state electronic timing device that allows to set up the operation time so that when the preset time is arrived at, the electric or mechanical power is outputted for the control of the controllable odor supply unit 101 to emit the odors for the session as preset;
-- an odor supply energy driver 103: to supply mechanical or electric power to the controllable odor supply unit 101 if the timing control device 102 is already provided with a local energy driver; or vice versa; and
-- a setting up device 104: related to a mechanical, dynamo-mechanical, or solid-state electronic device provided for setting up the controllable odor supply unit 101 to control the control timing of the timing control device 102 using the time telling unit of the prior art, or control the type of odor and the concentration of the olfactory odor emitted from timing control device 102, or set up the operation mechanisms of the controllable odor supply unit 101 and the timing control device 102 at the same time.

5. The timer controlled olfactory fragrance dispenser of Claim 4, wherein, the functional construction of telling the time by emitting olfactory odor of the time controlled olfactory odor dispenser described above functions as a clock of the prior art having the time telling measurement unit of the standard time telling device as the preset time telling unit so to control any or all of the following functions:
(1) Set up the controllable odor supply unit 101 to function as a clock in relation to the timing control device 102 for setting up the time telling operation using the time telling measurement unit of the standard time telling device as the unit to set up time telling; to control the source of olfactory odor to emit the same odor or different odors; or to control the intensity of the odor; or provide the session of continuous or intermittent emission of the olfactory odor;
(2) to set up point of time for the timing control device 102 to control the controllable odor supply unit 101 using the time telling measurement unit of the standard time telling device as the unit to set up time telling as described in subparagraph (1);
(3) Both functions respectively described in subparagraphs (1) and (2) are provided at the same time.

6. The timer controlled olfactory fragrance dispenser of Claim 4, wherein, in the time controlled olfactory odor dispenser functions as a clock of the prior art having the time telling measurement unit of the standard time telling device as the present time telling unit to provide the function of telling the time by emitting olfactory odor, an optional manual control device is adapted to the timer controlled olfactory fragrance dispenser for the purpose of controlling the controllable odor supply unit 101 to emit the odor or not and which type of odor to be emitted.

7. The timer controlled olfactory fragrance dispenser of Claim 4, wherein, the present invention functions as a traditional timer using the measuring unit of the standard time telling device to provide olfactory time telling function incorporated with conventional time telling function via sense of hearing and vision. Wherein, a conventional audio or video display unit 105 is further incorporated into the preferred embodiment while the timing device functions as a clock of the prior art and is set to be adapted with the time telling measurement unit of a standard time telling device using any of or any combination of the units including hour, half an hour, quarter, minute, second, day, night, month, season, and year to control the timing of the controllable odor supply unit 101 to emit olfactory odor for providing supplementary active time telling function in facilitating its use. Other than necessarily optional the casing and the general manual device, the preferred embodiment of the present invention is essentially comprised of the followings:
-- a controllable odor supply unit 101: comprised of one or more than one same or different olfactory odor supply sources in solid, liquid or gaseous state that are emitted either by itself, by heating, or by blowing, and is controlled by electric or mechanical power to be emitted into the designated space or not; an optional unit to set up and control the concentration of the odor may be disposed to the controllable odor supply unit 101;
-- a timing control device 102: related to a digital or analogy time output control device comprised of a mechanical, dynamo-mechanical or solid-state electronic timing device that allows to set up the operation time so that when the preset time is arrived at, the electric or mechanical power is outputted for the control of the controllable odor supply unit 101 to emit the odors for the session as preset;
-- an odor supply energy driver 103: to supply mechanical or electric power to the controllable odor supply unit 101 if the timing control device 102 is already provided with a local energy driver; or vice versa;
-- a setting up device 104: related to a mechanical, dynamo-mechanical, or solid-state electronic device provided for setting up the controllable odor supply unit 101 to control the control timing of the timing control device 102 using the time telling unit of the prior art, or control the type of odor and the concentration of the olfactory odor emitted from timing control device 102, or set up the operation mechanisms of the controllable odor supply unit 101 and the timing control device 102 at the same time; and
-- an audio or video display unit 105: related to audio device adapted to a timer comprised of conventional mechanical or electronic circuit including active time telling means including loudspeaker of a mechanical audio, or electronic device; or a video display device including a passive time telling device comprised of mechanical hands, jotting figures or electronic display.

8. The timer controlled olfactory fragrance dispenser of Claim 7, wherein, the functional construction of telling the time by emitting olfactory odor of the time controlled olfactory odor dispenser described above incorporated with conventional time telling functions of the conventionally audio and video time telling functions as a clock of the prior art having the time telling measurement unit of the standard time telling device as the preset time telling unit so to control any or all of the following functions:
(1) Set up the controllable odor supply unit 101 to function as a clock in relation to the timing control device 102 for setting up the time telling operation using the time telling measurement unit of the standard time telling device as the unit to set up time telling; to control the source of olfactory odor to emit the same odor or different odors; or to control the intensity of the odor; or provide the session of continuous or intermittent emission of the olfactory odor; and is at the same time provided with an audio device adapted to a timer comprised of conventionally mechanical or electronic circuit including active time telling means including loudspeaker of a mechanical audio, or electronic device; or a video display device including a passive time telling device comprised of mechanical hands, jotting figures or electronic display;
(2) to set up point of time for the timing control device 102 to control the controllable odor supply unit 101 using the time telling measurement unit of the standard time telling device as the unit to set up time telling as described in subparagraph (1);
(3) Both functions respectively described in subparagraphs (1) and (2) are provided at the same time.

9. The timer controlled olfactory fragrance dispenser of Claim 7, wherein, for the functional construction of telling the time by emitting olfactory odor of the time controlled olfactory odor dispenser incorporated with conventional time telling functions of the conventionally audio and video time telling, an optional manual control device is adapted to the timer controlled olfactory fragrance dispenser for the purpose of controlling the controllable odor supply unit 101 to emit the odor or not and which type of odor to be emitted.

10. The timer controlled olfactory fragrance dispenser of Claim 2, wherein, the time controlled olfactory odor supply device of the present invention within the scope of its basic working principles may be provided in desktop, wall mounting or portable fragrance dispenser adapted to a timer or not.

11. The timer controlled olfactory fragrance dispenser of Claim 4, wherein, the time controlled olfactory odor supply device of the present invention within the scope of its basic working principles may be provided in desktop, wall mounting or portable fragrance dispenser adapted to a timer or not.

12. The timer controlled olfactory fragrance dispenser of Claim 7, wherein, the time controlled olfactory odor supply device of the present invention within the scope of its basic working principles may be provided in desktop, wall mounting or portable fragrance dispenser adapted to a timer or not.
